# EUROPEAN PATENT SPECIFICATION

(11) **EP 3 907 510 B1**
(45) Date of publication and mention of the grant of the patent: **26.03.2025**
(21) Application number: 19906794.3
(22) Date of filing: 03.01.2019
(51) Int. Cl.: G01N 33/92, G01N 33/49

(54) **BIOCHEMICAL DIAGNOSTIC TEST FOR ATTENTION-DEFICIT/HYPERACTIVITY DISORDER (ADHD)**
BIOCHEMISCHER DIAGNOSTISCHER TEST FÜR AUFMERKSAMKEITSDEFIZIT/HYPERAKTIVITÄTSSTÖRUNG (ADHD)
TEST DIAGNOSTIQUE BIOCHIMIQUE POUR TROUBLE DÉFICIT DE L'ATTENTION/HYPERACTIVITÉ (TDAH)

(43) Date of publication of application: 10.11.2021
(73) Proprietor: Pontificia Universidad Católica De Chile, 8331150 Santiago (CL)
(72) Inventor: HENRÍQUEZ, Marcela, Santiago, 8331150 (CL); SOLARI, Sandra, Santiago, 8331150 (CL); QUIROGA, Sara, Santiago, 8331150 (CL); VARGAS, Sergio, Santiago, 8331150 (CL); ROJAS, Eric, Santiago, 8331150 (CL)
(74) Representative: Elzaburu S.L.P.
(86) International application number: PCT/CL2019/050002
(87) International publication number: WO 2020/140161

(56) References cited:
- WO-A1-2013/059669
- US-A1- 2012 094 315
- MARCELA P. HENRíQUEZ-HENRíQUEZ ET AL: "Low serum sphingolipids in children with attention deficit-hyperactivity disorder", FRONTIERS IN NEUROSCIENCE, vol. 9, 25 August 2015 (2015-08-25), pages 1 - 9, XP055558711, DOI: 10.3389/fnins.2015.00300
- TESEI ALESSANDRA ET AL: "The potential relevance of docosahexaenoic acid and eicosapentaenoic acid to the etiopathogenesis of childhood neuropsychiatric disorders", EUROPEAN CHILD AND ADOLESCENT PSYCHIATRY, HOGREFE & HUBER PUBLISHERS, BERN, CH, vol. 26, no. 9, 17 December 2016 (2016-12-17), pages 1011 - 1030, XP036315442, ISSN: 1018-8827, [retrieved on 20161217], DOI: 10.1007/S00787-016-0932-4
- GOW R V ET AL: "Total red blood cell concentrations of @w-3 fatty acids are associated with emotion-elicited neural activity in adolescent boys with attention-deficit hyperactivity disorder", PROSTAGLANDINS LEUKOTRIENES AND ESSENTIAL FATTY ACIDS, CHURCHILL LIVINGSTONE, EDINBURGH, vol. 80, no. 2-3, 1 February 2009 (2009-02-01), pages 151 - 156, XP025974220, ISSN: 0952-3278, [retrieved on 20090220], DOI: 10.1016/J.PLEFA.2008.12.007
- YONEZAWA KEN ET AL: "Investigation into the plasma concentration of [omega]3 polyunsaturated fatty acids in Japanese attention-deficit hyperactivity disorder patients", JOURNAL OF NEURAL TRANSMISSION, SPRINGER VIENNA, VIENNA, vol. 125, no. 9, 20 June 2018 (2018-06-20), pages 1395 - 1400, XP036569091, ISSN: 0300-9564, [retrieved on 20180620], DOI: 10.1007/S00702-018-1895-Z
- HENRIQUEZ-HENRIQUEZ, M. P. ET AL.: "Low serum sphingolipids in children with attention deficit-hyperactivity disorder", FRONTIERS IN NEUROSCIENCE, vol. 9, 2015, pages 300, XP026624203
- AGOSTONI, C. ET AL.: "The role of omega-3 fatty acids in developmental psychopathology: a systematic review on early psychosis, autism, and ADHD", INTERNATIONAL JOURNAL OF MOLECULAR SCIENCES, vol. 18, no. 12, 2017, pages 2608, XP055723115
- STEVENS, L. J. ET AL.: "Essential fatty acid metabolism in boys with attention-deficit hyperactivity disorder", THE AMERICAN JOURNAL OF CLINICAL NUTRITION, vol. 62, no. 4, 1995, pages 761 - 768, XP003023958
- SCHUCHARDT, J. P . ET AL.: "Significance of long-chain polyunsaturated fatty acids (PUFAs) for the development and behaviour of children", EUROPEAN JOURNAL OF PEDIATRICS, vol. 169, no. 2, 2010, pages 149 - 164, XP019781552
- BONVICINI, C. ET AL.: "Attention-deficit hyperactivity disorder in adults: a systematic review and meta-analysis of genetic, pharmacogenetic and biochemical studies", MOLECULAR PSYCHIATRY, vol. 21, no. 7, 2016, pages 872, XP037217058
- YOUNG, G. S. ET AL.: "Blood phospholipid fatty acid analysis of adults with and without attention deficit/hyperactivity disorder", LIPIDS, vol. 39, no. 2, 2004, pages 117 - 123, XP055723118

## Description

### Field of invention

The invention relates to a biochemical diagnostic test performed on peripheral blood, which allows the diagnosis of Attention Deficit Hyperactivity Disorder (ADHD). Specifically, the serum levels of at least 6 markers corresponding to 2 groups of functional lipids, sphingolipids and long-chain polyunsaturated fatty acids (LC-PUFAs) are measured, detecting a variation in concentrations with respect to the values of the control population.

### Background of the invention

ADHD is the most common neuro-psychiatric disorder in childhood, with global prevalences between 5-10%. The last decade has seen a marked increase in the diagnosis of ADHD and in the use of pharmacological treatment, which doubled between 2004 and 2015 (Davidovitch et al. BMC Pediatrics, 2017. 17:218). Since ADHD affects key areas in a child's intellectual, emotional, and relational development, the disorder is associated with a significant worsening in quality of life (Coghill et al. Eur Child Adolesc Psychiatry, 2017. 26:1283-1307).

Timely diagnosis and treatment of ADHD improves the patient's quality of life and mitigates the emotional and social impact of the disorder. However, pharmacological treatment is associated with increased cardiovascular risk (Shin et al. BMJ. 2016; 353: i2550.), therefore, diagnostic certainty is essential to assess the need for treatment. Currently, this diagnosis is exclusively clinical and based on the objectification of symptoms that meet the criteria established by consensus (DSM-5 or ICD-10), which require the doctor to assess the impact of the clinical picture on the social, school and emotional life of the child; an extremely difficult task in the context of medical consultations of an average duration between 15 and 30 minutes worldwide. Additionally, physicians face high comorbidity and clinical variability, which confuses diagnosis, and variability in symptomatic assessment from different reporting sources (McKeown et al. JAm Acad Child Adolesc Psychiatry, 2015 54(1): 53-6). All of this makes diagnosing ADHD a major clinical challenge, requiring in most cases several evaluations by a well-trained physician. Currently the gold standard for the diagnosis of this disorder is the clinical diagnosis made by a doctor specializing in child neurology/psychiatry, supported by neuropsychological studies that - although they have a very limited diagnostic value - help to characterize the specific cognitive profile for the patient and to rule out other alterations.

In this way the diagnosis of ADHD is exclusively clinical and does NOT have the diagnostic support of any objective tool. There is no specific neuropsychological profile for ADHD, so the application of batteries of neurocognitive tests has limited diagnostic utility. Having an objective diagnostic tool for ADHD would simplify and shorten the diagnostic process, reduce diagnostic errors and improve adherence to treatment.

So far, attempts to have an objective diagnostic tool for ADHD have focused mostly on the development of neuro-imaging-based detection algorithms, which have achieved sensitivities and specificities that rarely exceed 80% (Uddin et al. Transl Psychiatry, 2017; 7, e1218). Although some of these methods show promising results, their clinical applicability is limited in children with ADHD, due to the low acceptability of the technique in these patients (Yerys et al. Hum Brain Mapp, 2009; 30:3426-3435). Additionally, extending the use of neuroimaging to isolated regions or large patient populations (as in a screening) is technically and economically difficult. The use of electro-encephalographic markers for the detection of ADHD has also been explored, with good results, the main limitation of these techniques being the low reproducibility of some of the analyses used (for example, ERPs; Kompatsiari et al. Neuroscience Letters, 2016; 617: 166-172).

Some groups of researchers, including one from the medical school of Universidad Católica de Chile, have reported algorithms based on the characterization of eye movements or pupillary size (Varela Casal et al. Journal of Attention Disorders, 2018; 1-16; Wainstein et al, Scientific Reports, 2017; 7: 8228). The use of these latter algorithms is attractive since they do not require invasive procedures and their implementation is low cost, but they need specific equipment, which limits their widespread application.

Previously, some research groups have explored the use of biochemical markers for the diagnosis of ADHD, for example Vogel et al. (Psychoneuro-endocrinology, 2017; 79:31-39), Alassehirli et al (The International Journal of Psychiatry in Medicine, 2015; 50(2): 238-247) and Percinel et al. (Child Psychiatry Hum Dev, 2016; 47:259-269), however its results are not very replicable and none has achieved the levels of sensitivity and specificity of those obtained by the method of this invention.

The need therefore persists for an objective analytical diagnostic test of easy implementation and interpretation that is a tool to support the current clinical diagnosis.

The inventors have met this need by developing a biochemical diagnostic test which, surprisingly, allows the diagnosis of Attention Deficit Hyperactivity Disorder by analyzing peripheral blood of patients.

### DESCRIPTION OF THE FIGURES

**Figure 1****.**

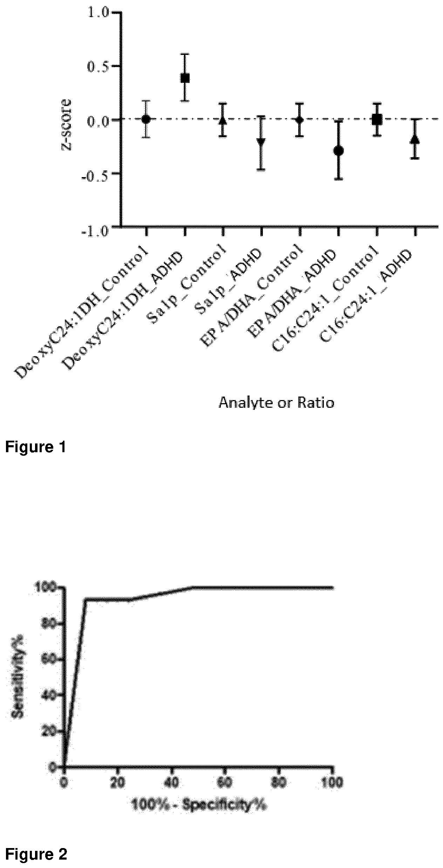

   Figure 1 shows the variation of the score (z-score) obtained for each variable considered in the method of the invention for the control population and for the group with ADHD. The z-score represents the distance between the mean value and the mean of the reference population, in standard deviations. That is, the figure shows how many standard deviations above/below the mean of the control group is the mean of children with ADHD. The control group determines the value 0.
**Figure2****.**
   Figure 2 shows the ROC curve of the method of the invention for 77 cases analyzed (AUC = 0.95 p<0.001), Sensitivity 93.1%, and Specificity 84%.

### DESCRIPTION OF THE INVENTION.

The invention aims at a biochemical diagnostic test performed on peripheral blood, which allows the diagnosis of Attention Deficit Hyperactivity Disorder (ADHD), with sensitivity of 93% and specificity of 84% or higher according to pilot results. The sensitivity and specificity indicated were evaluated in relation to a gold standard compound, widely used in research, comprising the clinical diagnosis and the positive response of the patient to the specific pharmacological treatment of the disease.

In a single aspect, the invention relates to an In vitro diagnostic method of ADHD characterized in that it comprises detecting from a peripheral blood sample the variation in normal serum levels in relation to the mean of a healthy reference population of the following lipids: Ceramide C16:0, Ceramide C24:0, Deoxy dihydro-ceramide C24:1 and Sphinganin 1-phosphate, docosahexaenoic acid (DHA) and eicosapentaenoic acid (EPA); and determining if there is a variation in the absolute values for Deoxy dihydro-ceramide C24:1 and Sphinganin 1-P, respect to the normal values obtained in the healthy population control group; determining if there is a variation in the ratio Ceramide C24:0/Ceramide C16:0 (C24:0/C16:0) and DHA/EPA, respect to the normal values obtained in the healthy population control group; and classifying the sample as positive for ADHD if there is an increase in the absolute value of Deoxy dihydro-ceramide C24:1; and/or a decrease in the absolute value for Sphinganin 1-P; and/or a decrease in the value of the C24:0/C16:0 ratio and/or DHA/EPA, with respect to the normal values obtained in the healthy population control group.

The biochemical test of the invention is based on the analysis of serum levels of 6 markers, corresponding to 2 groups of functional lipids, sphingolipids and long-chain polyunsaturated fatty acids (LC-PUFAs), detecting a variation in the concentrations of said markers with respect to the values for the control population. Specifically, the markers used are the sphingolipids Ceramide C16:0, Ceramide C24:0, Deoxy dihydro-ceramide C24:1 and Sphinganine 1-phosphate and the LC-PUFAs docosahexaenoic acid (DHA) and eicosapentaenoic acid (EPA).

The invention comprises 2 consecutive phases: a first phase of analytical processing and a second phase of statistical processing. In the first phase, serum levels of at least 6 markers corresponding to 2 groups of functional lipids, sphingolipids and long-chain polyunsaturated fatty acids (LC-PUFAs) are measured, and up to 28 functional lipid markers can be measured, which would contribute to obtaining greater specificity in the results while maintaining predictive quality.

The markers are measured preferably in serum obtained by centrifugation of a peripheral blood sample collected in a tube without anticoagulant, by LC-MSMS after lipid extraction, in the case of sphingolipids, or by GC-MS after extraction and derivatization of lipids, in the case of LC-PUFAs. Both technologies are considered the gold standard for the analytical determination of each group of markers.

In the second phase, whether patients are positive or negative for ADHD is determined using a predictive algorithm developed by the inventors that receives as input variables the serum levels of the indicated markers, normalizes the data and confers a total score to each sample, which is submitted to a critical decision point to obtain the classification. Depending on the selected markers, the absolute value of each marker or a ratio between two markers will be used. Due to its high sensitivity, the most appropriate use of this test is diagnostic screening.

The method, as indicated, is based on the joint determination of 6 lipid markers, which are used as input variables for a predictive algorithm. The joint use of these markers to give greater sensitivity and specificity to ADHD discrimination is new and not obvious, given that no significant differences in ADHD have been reported for some of them, such as sphinganine 1-phosphate. This reveals that the relative balance of the levels of these markers - and not their absolute level - gives the discriminating power to the method of the invention.

Optionally the method can use as input variables up to 28 lipid markers, selected from sphinganin-1-phosphate, sphingosine-1-phosphate, ceramides C16:0, C18:0, C20:0, C22:0, C24:0 and C24:1; dihidroceramides C18, C18:1, C24:0 and C24:1; deoxi- ceramides C16:0 and C24:1 and deoxi-dihidroceramides C16:0 and C24:1; sphingomyelins (SM) SM C16:0, SM C18:0, SM C18:1 and SM C24:1, linoleic acid (LA), •-linoleic acid (GLA), alpha-linolenic acid (ALA), dihomo-gamalinolenic acid (DGLA), arachidonic acid (AA), eicosapentaenoic acid (EPA), docosapentanoic acidor (DPA), docosahexaenoic acid (DHA).

In the case of opting for a complex diagnostic test, with a greater number of variables, the classification model obtained demonstrated a sensitivity of 93% and a specificity of 96% in the training procedure of the predictive algorithm and a sensitivity of 100% and specificity of 83% during the validation procedure of the predictive algorithm.

So far there is no validated tool that fulfills the function of the method proposed by this invention. There are publications of algorithms based on neuroimaging (NMR) and EEG for the detection of ADHD, which -in general- do not present sensitivities/specificities greater than 80% and show little analytical reproducibility, in addition to the complexity of implementation. Since the analytical phase of our diagnostic test is performed using techniques that constitute the gold standard for each group of molecules, the analytical imprecision associated with it is the least possible with current technology (in general less than 10% inter-run). Finally, the use of a peripheral biological sample, minimally invasive and easy to store and transport, gives this test comparative advantages in relation to NMR or EEG for widespread use, for example, as a screening tool.

Recently the inventors published for the first time the significant decrease of specific sphingolipids in children with ADHD (especially sphingomyelins and long-chain ceramides) and raised the importance of this group of functional lipids as an eventual biomarker and/or pathogenic factor for ADHD. These results are based on a pilot study that characterized the serum levels of 20 molecular species of sphingolipids in 90 subjects, including patients with ADHD and 2 control groups: a) first-degree relatives without diagnosis of ADHD who live and share their main meal with the index case and b) controls without ADHD and without family history of the disease. This pilot study described that low serum levels of total sphingomyelins present a sensitivity of 79% and specificity of 80% for the diagnosis of ADHD (Henríquez-Henríquez et al. Front. Neurosci, 2015. 9:300).

In a subsequent analysis of the data obtained, the inventors were discriminating the markers that correlate with the development of ADHD, finding that the combination of markers of the 2 lipid families (sphingolipids and LC-PUFAs) are able to discriminate patients with ADHD more efficiently than that reported by the same group in the pilot study. The results of the analysis of ROC on the classification achieved by the method of the invention demonstrate that it is able to discriminate patients with ADHD with a sensitivity and specificity much higher than that reported in the previous publication (sensitivity 93%, specificity 84%).

For the expert in the technique, it will be evident that each of the markers can be detected with any method available in the technique, as the method of the invention correlates the variation of normal values with the presence of attention deficit and hyperactivity disorder. For this reason, the invention can be applied independently of the technique by which the concentration of the selected lipids is determined and regardless of the number of markers used. All these embodiments are considered within the scope of the present invention.

Thus, as indicated by the in vitro diagnostic method for Attention Deficit Hyperactivity Disorder (ADHD) comprises a first phase of analytic measurement from a peripheral blood sample of the markers selected from 2 groups of functional lipids: sphingolipids and long-chain polyunsaturated fatty acids (LC-PUFAs) and a second statistical phase where the normalized value for each marker is determined, assigning a total score to the sample using a predictive model and classifying it as positive or negative for ADHD if said total score exceeds an established value.

The markers selected in the first phase correspond to at least 6 functional lipids to achieve diagnostic accuracy, and up to 28 markers can be measured to obtain a greater sensitivity/specificity in the results. Depending on the set of markers used, the variables to be evaluated may be absolute values and/or a ratio between markers.

The 6 markers determined in the first stage determine 4 variables for the statistical phase, since 4 of them are analyzed as ratios.

The score is assigned by calculating the z-score for each variable analyzed, considering as an average value the amount obtained for the control population without ADHD determined according to clinical criteria (gold standard). The z-score obtained for each marker or marker ratio is used as an input variable in the predictive model that will determine the score.

A sample will be considered ADHD positive if the score obtained in the predictive algorithm is less than an established value of critical decision, or in other words if there is a minimum set of differences in the variables analyzed with respect to the normal values obtained in the control group for these same variables.

In the case of the measurement of 6 markers for the use of 4 variables (see Figure 1), the sample will be classified as positive for ADHD if there is a critical difference between the following variations in the sample studied with respect to the normal values obtained in the control group:
- an increase in the absolute value of Deoxy dihydro-ceramide C24:1 and/or
- a decrease in the absolute value for Sphinganin 1-P; and/or
- a decrease in the value of the Ceramide C24:0/Ceramide C16:0 ratio and/or the DHA/EPA ratio.

In a preferred embodiment, the score of the diagnostic method is calculated after applying a predictive model on the input variables (z-score) representing the variation of the absolute values for Deoxy dihydro-ceramide C24:1 and Sphinganin 1-P and ratio Ceramide C24:0/Ceramide C16:0 (C24:0/C16:0) and DHA/EPA with respect to the normal values obtained in the healthy population control group.

Where the critical difference is determined in relation to the score assigned by a predictive algorithm, which will relate the existence/non-existence of these variations in the studied sample with respect to the normal values obtained in the control group. And where the critical decision point is established by correlating the values obtained in samples of patients without ADHD and samples of patients with ADHD, for a given population.

This can be done with the 4 variables corresponding to the 6 main markers, and optionally with one or more of these 22 markers: sphingosine-1-phosphate, ceramides C18:0, C20:0, C22:0 and C24:1; dihydroceramides C18, C18:1, C24:0 and C24:1; deoxy-ceramides C16:0 and C24:1 and deoxy-dihydroceramide C16:0; sphingomyelins (SM) SM C16:0, SM C18:0, SM C18:1 and SM C24:1, linoleic acid (LA), •-linoleic acid (GLA), alpha-linolenic acid (ALA), dihomo-gamalinolenic acid (DGLA) arachidonic acid (AA), and docosapentanoic acid (DPA).

Thus, in a preferred embodiment, the 28 markers are studied and the diagnostic method is based in that in addition to the 6 lipids are optionally incorporated one or more of these 22 markers: sphingosine-1-phosphate, ceramides C18:0, C20:0, C22:0 and C24:1; dihydroceramides C18, C18:1, C24:0 and C24:1; deoxy-ceramides C16:0 and C24:1 and deoxy-dihydroceramide C16:0; sphingomyelins (SM) SM C16:0, SM C18:0, SM C18:1 and SM C24:1, linoleic acid (LA), •-linoleic acid (GLA), alpha-linolenic acid (ALA), dihomo-gamalinolenic acid (DGLA), arachidonic acid (AA), and docosapentanoic acid (DPA), to obtain greater diagnostic specificity.

In the case of using the optional method with 6 or up to 28 markers, once the serum values of each one have been determined, a correlation is made between the variation of serum concentrations and the clinical diagnosis of ADHD. This assigns a score to even small differences in a set of variables. Thus, a sample is generally considered as ADHD positive if the score obtained in a predictive algorithm differs from the established value of critical decision, where the algorithm determines the minimum conditions for the classification to be positive. These 22 markers can be evaluated by their absolute values or as ratios between 2 of them, for a pair of markers.

Thus, in a preferred embodiment, for each new marker analyzed, a variable is established, whose score is calculated after applying a predictive model to the input variables (z-score) that represents the variation of the absolute values of said marker with respect to the normal values obtained in the healthy population control group.

In a preferred embodiment, for two of these new markers analyzed in pairs, a variable is established, whose score is calculated after applying a predictive model on the input variables (z-score) representing the variation of the values in the ratio of said pair of markers with respect to the normal values obtained in the healthy population control group.

The predictive algorithm can be developed using various analytical tools, including mathematical or statistical modeling and machine learning (neural networks, etc.).

Next, a preferred mode of embodiments of the invention is exemplified within the scope of the inventive concept that we protect in this invention.

### Example 1.

### 1.1 Blood sample collection and pre-analytical processing.

The sample is obtained by peripheral venous puncture, with minimum fasting of 4 hours, in a tube without anticoagulant. In the laboratory the sample is centrifuged to obtain serum, which passes to the analysis phase.

### 1.2 Measurement of Lipidomic Markers.

For the analysis of sphingolypid markers, serum lipids are extracted, using a technique standardized in our laboratory and widely validated by the literature (Folch's modified method, Folch J., Lees M., Sloane-Stanley G.H. A simple method for the isolation and purification of total lipids from animal tissues. J. Biol. Chem. 1957;226:497-509). Sphingolipids are then detected and quantified using a previously standardized protocol on a liquid chromatography - LC-MS/MS mass spectrometry platform. For the analysis of the markers corresponding to LC-PUFAs, the serum lipids are extracted and derivatized by protocol and the LC-PUFAs are detected and quantified by means of a standardized protocol in gas chromatography - GC-MS mass spectrometry platform. The analytical features of this last protocol have been published by our laboratory (Henriquez-Henriquez et al. • -3 Long-Chain Polyunsaturated Fatty Acids and Fatty Acid Desaturase Activity Ratios as Eventual Endophenotypes for ADHD. J Atten Disord. 2015 Nov;19(11):977-86.doi:10.1177/1087054712461175).

### 1.3 Application of The Classificatory Model.

In the second phase, a predictive model receives as input variables the serum levels of the 6 markers indicated, either as an absolute marker or as a ratio (absolute value for Deoxy dihydro-ceramide C24:1 and Sphinganin 1-P and ratio C24:0/C16:0 and DHA/EPA). The method was validated considering training and validation data, and then proceeded to execute the classification with each of the samples included in the study data. Through an algorithm, each sample is assigned a total score or score, which is subjected to a critical decision point to obtain the classification that allows determination of whether the sample is positive or negative for ADHD.

The differences that exist for the different analytes of patients with ADHD and the healthy population, without this disorder, were determined. The inventors found that there are differences between the serum concentrations of Deoxy dihydro-ceramide C24:1 and Sphinganin 1-P between the two groups and that the difference between the ratios of Ceramide C24:0/Ceramide C16:0 is also relevant (C24:0/C16:0) and DHA/EPA. The results are shown in Figure 1, which compares the average value of the healthy population for each variable, marker or ratio, assigning it the value or z-score 0, and the standard deviations of the average found in these variables for the population with ADHD. It is clearly seen that in the group with ADHD there is an increase in the serum concentration of Deoxy dihydro-ceramide C24:1, while a decrease is seen in the serum concentration of Sphinganin 1-P and the ratios of the concentrations of Ceramide C24:0/Ceramide C16:0 (C24:0/C16:0) and DHA/EPA with respect to the normal values obtained in the control group.

### Example 2.

The serum values of the markers of the invention were analyzed in samples from 11 patients, between 7 and 17 years of age, 6 of them with clinical diagnosis of ADHD and 5 healthy controls. A correlation was made between the variation of serum concentrations of Ceramide C16:0, Ceramide C24:0, Deoxy dihydro-ceramide C24:1, Sphinganin 1-phosphate, docosahexaenoic acid (DHA) and eicosapentaenoic acid (EPA) and the clinical diagnosis of ADHD.

The results are shown in Table 1, which details the absolute levels obtained in peripheral blood for the 6 analytes considered in the invention, and the value of the score or score assigned by its variation, of the analyte itself for Deoxy dihydro-ceramide C24:1 and Sphinganin 1-P or the ratios already indicated, C24:0/C16:0 and DHA/EPA. The cut-off point (or critical decision point) was designated in the score 0.5, where a score less than or equal to 0.5 is indicative of ADHD, and a score greater than 0.5 is indicative of the absence of the disease. In these 11 cases chosen at random, the method of the invention had a concordance of 82% (9 of 11) with the composite diagnosis or *gold standard.*

In this table Deoxy dihydro-ceramide C24:1 is identified as DeoxyC24:1DH, Sphinganin 1-P is identified as Sa1p., Ceramide C16:0 is identified as C16, Ceramide C24:0 is identified as C24, docosahexaenoic acid is identified as DHA and eicosapentaenoic acid is identified as EPA.

In the analyzed data it can be seen that there were 2 cases where one of the lipids, specifically Sphinganine 1-P (Sa1p) was not determined. As the method of the invention weights the different variations of the 6 analytes, it is possible to use it even without one of them, and as you can see in the results, the method maintains its diagnostic predictive value.

**Table 1. 0= ADHD -, 1= ADHD +, nd= not determined**

| case | ADHD Diagnosis | C16 µM | Deoxy C24:1DH µM | Sa1p µM | C24 µM | Epa µM | Dha µM | score | Invention Method | Gold Standard Concordance |
|---|---|---|---|---|---|---|---|---|---|---|
| 1 | 0 | 0.130 | 0.0205 | nd | 2.3390 | 9.47 | 48.42 | 0.552 | 0 | Yes |
| 2 | 1 | 0.187 | 0.0324 | 0.2389 | 2.1552 | 245.92 | 167.13 | 0.489 | 1 | Yes |
| 3 | 1 | 0.117 | 0.0112 | 0.1421 | 1.4071 | 6.99 | 31.31 | 0.161 | 1 | Yes |
| 4 | 1 | 0.150 | 0.0258 | 0.2484 | 1.5269 | 8.62 | 37.47 | 0.279 | 1 | Yes |
| 5 | 0 | 0.167 | 0.0262 | 0.1802 | 1.9104 | 17.55 | 51.34 | 0.691 | 0 | Yes |
| 6 | 1 | 0.149 | 0.0149 | 0.2341 | 1.3953 | 12.87 | 58.55 | 0.324 | 1 | Yes |
| 7 | 0 | 0.128 | 0.0070 | 0.1388 | 1.5624 | 9.51 | 37.94 | 0.410 | 1 | No |
| 8 | 1 | 0.113 | 0.0119 | nd | 2.0165 | 5.63 | 19.57 | 0.116 | 1 | Yes |
| 9 | 0 | 0.142 | 0.0196 | 0.2209 | 1.6027 | 8.19 | 20.31 | 0.361 | 1 | No |
| 10 | 0 | 0.209 | 0.0224 | 0.1239 | 1.7728 | 17.66 | 61.67 | 0.822 | 0 | Yes |
| 11 | 1 | 0.171 | 0.0115 | 0.1357 | 2.5371 | 12.75 | 52.54 | 0.189 | 1 | Yes |

### Example 3.

The serum values of the markers of the invention were analyzed in samples from 77 patients, 28 of them with clinical diagnosis of ADHD and 49 healthy controls. A correlation was made between the variation of serum concentrations of Ceramide C16:0, Ceramide C24:0, Deoxy dihydro-ceramide C24:1, Sphinganin 1-phosphate, docosahexaenoic acid (DHA) and eicosapentaenoic acid (EPA) and the clinical diagnosis of ADHD. The results are shown in Figure 2, where a ROC chart can be seen, with an area under the curve of 0.95. For a ROC analysis the area under the curve is directly correlated with the accurate diagnosis, with an area under the curve with a value of 1 being a perfect diagnosis. In this case, the area under the curve has a value of 0.95, this means that there is a 95% probability that the test allows the diagnosis of the pathology analyzed, which corresponds to ADHD.

These results demonstrate the great usefulness of the test of the invention as an objective tool for the diagnosis of ADHD.

## Claims

1. In vitro diagnostic method of Attention Deficit Hyperactivity Disorder (ADHD) **CHARACTERIZED in that** it comprises:
• detecting from a peripheral blood sample the variation in normal serum levels in relation to the mean of a healthy reference population of the following lipids: Ceramide C16:0, Ceramide C24:0, Deoxy dihydro-ceramide C24:1 and Sphinganin 1-phosphate, docosahexaenoic acid (DHA) and eicosapentaenoic acid (EPA); and
• determining if there is a variation in the absolute values for Deoxy dihydro-ceramide C24:1 and Sphinganin 1-P, respect to the normal values obtained in the healthy population control group;
• determining if there is a variation in the ratio Ceramide C24:0/Ceramide C16:0 (C24:0/C16:0) and DHA/EPA, respect to the normal values obtained in the healthy population control group; and
• classifying the sample as positive for ADHD if there is:
∘ an increase in the absolute value of Deoxy dihydro-ceramide C24:1; and/or
∘ a decrease in the absolute value for Sphinganin 1-P; and/or
∘ a decrease in the value of the C24:0/C16:0 ratio and/or DHA/EPA,
with respect to the normal values obtained in the healthy population control group.

2. Diagnostic method according to claim 1, **CHARACTERIZED in that** the score is calculated after applying a predictive model on the input variables (z-score) representing the variation of the absolute values for Deoxy dihydro-ceramide C24:1 and Sphinganin 1-P and ratio Ceramide C24:0/Ceramide C16:0 (C24:0/C16:0) and DHA/EPA with respect to the normal values obtained in the healthy population control group.

3. Diagnostic method according to claim 1, **CHARACTERIZED in that** in addition to the 6 lipids one or more of these 22 markers are identified: sphingosine-1-phosphate, ceramides C18:0, C20:0, C22:0 and C24:1; dihydroceramides C18, C18:1, C24:0 and C24:1; deoxy-ceramides C16:0 and C24:1 and deoxy-dihydroceramide C16:0; sphingomyelins (SM) SM C16:0, SM C18:0, SM C18:1 and SM C24:1, linoleic acid (LA), γ-linoleic acid (GLA), alpha-linolenic acid (ALA), dihomo-gamalinolenic acid (DGLA), arachidonic acid (AA), and docosapentanoic acid (DPA), to obtain greater diagnostic specificity.

4. Diagnostic method according to claim 3, **CHARACTERIZED in that** for each new marker analyzed, a variable is established, whose score is calculated after applying a predictive model to the input variables (z-score) that represents the variation of the absolute values of said marker with respect to the normal values obtained in the healthy population control group.

5. Diagnostic method according to claim 3, **CHARACTERIZED in that** for two of these new markers analyzed in pairs, a variable is established, whose score is calculated after applying a predictive model on the input variables (z-score) representing the variation of the values in the ratio of said pair of markers with respect to the normal values obtained in the healthy population control group.

## Patentansprüche

1. In-vitro-Diagnoseverfahren der Aufmerksamkeitsdefizit-Hyperaktivitätsstörung (ADHS), **DADURCH GEKENNZEICHNET, dass** es Folgendes umfasst:
• erfassen anhand einer peripheren Blutprobe der Variation im normalen Serumspiegel im Verhältnis zum Mittelwert einer gesunden Referenzpopulation der folgenden Lipide: Ceramid C16:0, Ceramid C24:0, Deoxydihydroceramid C24:1 und Sphinganin-1-phosphat, Docosahexaensäure (DHA) und Eicosapentaensäure (EPA); und
• bestimmen, ob eine Abweichung der absoluten Werte für Deoxy-Dihydro-Ceramid C24:1 und Sphinganin 1-P in Bezug auf die in der Kontrollgruppe der gesunden Bevölkerung erhaltenen Normalwerte vorliegt;
• bestimmen, ob es eine Variation des Verhältnisses von Ceramid C24:0/Ceramid C16:0 (C24:0/C16:0) und DHA/EPA in Bezug auf die in der gesunden Bevölkerungskontrollgruppe erhaltenen Normalwerte gibt; und
• einstufen der Probe als positiv für ADHS, wenn:
∘ eine Erhöhung des absoluten Wertes von Deoxy-Dihydro-Ceramid C24:1; und/oder
∘ eine Abnahme des absoluten Werts für Sphinganin 1 -P; und/oder o
∘ eine Abnahme des Wertes des C24:0/C16:0-Verhältnisses und/oder DHA/EPA,
in Bezug auf die Normalwerte, die in der Kontrollgruppe der gesunden Bevölkerung erhalten wurden, vorliegt.

2. Diagnoseverfahren nach Anspruch 1, **DADURCH GEKENNZEICHNET, dass** der Score nach Anwendung eines prädiktiven Modells auf die Eingangsvariablen (z-Score) berechnet wird, das die Variation der Absolutwerte für Deoxy-Dihydro-Ceramid C24:1 und Sphinganin 1-P und das Verhältnis von Ceramid C24:0/Ceramid C16:0 (C24:0/C16:0) und DHA/EPA in Bezug auf die in der gesunden Populationskontrollgruppe erhaltenen Normalwerte darstellt.

3. Diagnoseverfahren nach Anspruch 1, **DADURCH GEKENNZEICHNET, dass** zusätzlich zu den 6 Lipiden einer oder mehrere dieser 22 Marker identifiziert werden; Sphingosin-1-phosphat, Ceramide C18:0, C20:0, C22:0 und C24:1; Dihydroceramide C18, C18:1, C24:0 und C24:1; Desoxy-Ceramide C16:0 und C24:1 und Desoxy-Dihydroceramid C16:0; Sphingomyeline (SM) SM C16:0, SM C18:0, SM C18:1 und SM C24:1, Linolsäure (LA), Y-Linolsäure (GLA), Alpha-Linolensäure (ALA), Dihomogamalinolensäure (DGLA), Arachidonsäure (AA) und Docosapentansäure (DPA), um eine größere diagnostische Spezifität zu erhalten.

4. Diagnoseverfahren nach Anspruch 3, **DADURCH GEKENNZEICHNET, dass** für jeden neuen analysierten Marker eine Variable festgelegt wird, deren Score nach Anwendung eines prädiktiven Modells auf die Eingangsvariablen (z-Score) berechnet wird, das die Variation der Absolutwerte des Markers in Bezug auf die in der gesunden Populationskontrollgruppe erhaltenen Normalwerte darstellt.

5. Diagnoseverfahren nach Anspruch 3, **DADURCH GEKENNZEICHNET, dass** für zwei dieser paarweise analysierten neuen Marker eine Variable festgelegt wird, deren Punktzahl nach Anwendung eines prädiktiven Modells auf die Eingangsvariablen (z-Score) berechnet wird, das die Variation der Werte im Verhältnis des Paars von Markern zu den Normalwerten darstellt, die in der Kontrollgruppe der gesunden Population erhalten wurden.

## Revendications

1. Méthode de diagnostic in vitro du trouble de déficit de l'attention avec ou sans hyperactivité (TDAH) **CARACTÉRISÉE en ce qu'**elle consiste à :
• détecter à partir d'un échantillon de sang périphérique la variation des taux sériques normaux par rapport à la moyenne d'une population saine de référence des lipides suivants : céramide C16:0, céramide C24:0, désoxy-dihydrocéramide C24:1 et sphinganine 1-phosphate, acide docosahexaénoïque (DHA) et acide eicosapentaénoïque (EPA) ; et
• déterminer s'il existe une variation des valeurs absolues pour le désoxy-dihydrocéramide C24:1 et la sphinganine 1-P, par rapport aux valeurs normales obtenues dans le groupe témoin de population saine ;
• déterminer s'il existe une variation dans le rapport céramide C24:0/céramide C16:0 (C24:0/C16:0) et DHA/EPA, par rapport aux valeurs normales obtenues dans le groupe témoin de population saine ; et
• classer l'échantillon comme positif au TDAH s'il y a :
∘ une augmentation de la valeur absolue du désoxy-dihydrocéramide C24:1 ; et/ou
∘ une diminution de la valeur absolue de la sphinganine 1-P ; et/ou
∘ une diminution de la valeur du rapport C24:0/C16:0 et/ou DHA/EPA,
par rapport aux valeurs normales obtenues dans le groupe témoin de population saine.

2. Méthode de diagnostic selon la revendication 1, **CARACTÉRISÉE en ce que** le score est calculé après application d'un modèle prédictif sur les variables d'entrée (score z) représentant la variation des valeurs absolues pour le désoxy-dihydrocéramide C24:1 et la sphinganine 1-P et le rapport céramide C24:0/céramide C16:0 (C24:0/C16:0) et DHA/EPA par rapport aux valeurs normales obtenues dans le groupe témoin de population saine.

3. Méthode de diagnostic selon la revendication 1, **CARACTÉRISÉE en ce qu'**en plus des 6 lipides, un ou plusieurs des 22 marqueurs suivants sont identifiés : sphingosine-1-phosphate, céramides C18:0, C20:0, C22:0 et C24:1 ; dihydrocéramides C18, C18:1, C24:0 et C24:1 ; désoxy-céramides C16:0 et C24:1 et désoxy-dihydrocéramide C16:0 ; sphingomyélines (SM) SM C16:0, SM C18:0, SM C18:1 et SM C24:1, acide linoléique (LA), acide y-linoléique (GLA), acide alpha-linolénique (ALA), acide dihomo-gamalinolénique (DGLA), acide arachidonique (AA) et acide docosapentanoïque (DPA), pour obtenir une plus grande spécificité diagnostique.

4. Méthode de diagnostic selon la revendication 3, **CARACTÉRISÉE en ce que** pour chaque nouveau marqueur analysé, une variable est établie, dont le score est calculé après application d'un modèle prédictif aux variables d'entrée (score z) représentant la variation des valeurs absolues dudit marqueur par rapport aux valeurs normales obtenues dans le groupe témoin de population saine.

5. Méthode de diagnostic selon la revendication 3, **CARACTÉRISÉE en ce que** pour deux de ces nouveaux marqueurs analysés par paires, une variable est établie, dont le score est calculé après application d'un modèle prédictif sur les variables d'entrée (score z) représentant la variation des valeurs dans le rapport de ladite paire de marqueurs par rapport aux valeurs normales obtenues dans le groupe témoin de population saine.
